# EUROPEAN PATENT APPLICATION

(11) **EP 1 133 999 A1**
(43) Date of publication of application: **19.09.2001**
(21) Application number: 00302071.6
(22) Date of filing: 15.03.2000
(51) Int. Cl.: A61L 15/46

(54) **Catechin containing sanitary goods**

(71) Applicant: Kabushiki Kaisha Uenoyahonpo, Onomichi-shi, Hiroshima-ken (JP)
(72) Inventor: Masutaka, Kake c/o Kabushiki Kaisha Uenoyahonpo, Onomichi-shi, Hiroshima-ken (JP)
(74) Representative: Allen, Philippa Margaret

(57) **Abstract**

Catechin containing sanitary goods. Catechin is extracted with purified water from tea leaves grown organically without agricultural chemicals in Sri Lanka. Such organic catechin is subjected to dextrin treatment. The sanitary goods are impregnated with the organic catechin. Accordingly, the sanitary goods are safe and superior in sterilization and disinfection effect.

## Description

### TECHNICAL FIELD

The present invention relates to catechin containing sanitary goods such as sanitary napkins, paper diapers, swabs, and so on.

### BACKGROUND ART

In the background art, there are sanitary goods containing catechin for the purpose of sterilization and disinfection. Unsatisfactorily, however, catechin used in such sanitary goods is extracted from tea leaves grown with agricultural chemicals, and alcohol is used for the catechin extraction.The inventor has surprisingly found that this organic soluble molecule can in fact be separated by water extraction using the specific procedure.

### SUMMARY OF THE INVENTION

It is an object of the present invention to provide sanitary goods which contain catechin extracted with purified water and which are superior in sterilization and disinfection effect in comparison with background-art catechin containing sanitary goods.

In order to attain the foregoing object, according to the present invention, organic catechin (grown organically without agricultural chemicals) is used so as to obtain catechin containing sanitary goods superior in sterilization and disinfection effect.

### DESCRIPTION OF THE EMBODIMENT OF THE INVENTION

An embodiment of the present invention will be described. Catechin containing sanitary goods in this embodiment have a feature that the sanitary goods are impregnated with liquid of organic catechin extracted with purified water from tea leaves grown organically without agricultural chemicals, such as tea leaves grown in Sri Lanka.

A proper quantity of Sri Lanka tea leaves (crude tea: black tea type prepared like green tea) is put into purified water and boiled. Thus, extracted material is extracted from the tea leaves. Further, the extracted material is centrifugally concentrated with thin film. Sanitary goods are coated with such material by spraying. However, the present invention is not limited to this manner.

Sri Lanka tea leaves are grown organically without agricultural chemicals. Catechin extracted from such tea leaves prepared like green tea is subjected to a dextrin treatment. That is, tea-leaf catechin is coated with cyclic oligosaccharide obtained by applying an enzyme (cyclodextrin glucanotranferase) to starch and containing glucose as its constituent unit. Because of such coating, the tea-leaf catechin is prevented from being oxidized. According to analysis and test result paper of Japan Food Research Laboratories, extracted liquid from Sri Lanka tea leaves was analyzed as follows.

**Table 1**

| analysis test item | result | detection limit |
|---|---|---|
| moisture | 4.4g/100g | |
| tannin | 43.2% | |
| anhydrous caffeine | 6.8g/100g | |
| arsenic (as As₂O₃) | not detected | 0.1ppm |
| lead | 16.2ppm | |
| cadmium | not detected | 0.01ppm |
| total mercury | not detected | 0.01ppm |
| number of general bacteria (number of living bacreria) | 300/g or less | |
| coliform group | negative/2.22g | |

According to the present invention, catechin is extracted from Sri Lanka tea leaves grown without agricultural chemicals, and subjected to dextrin treatment, and sanitary goods are impregnated with such organic catechin. Accordingly, the sanitary goods are safe because the catechin contains no agricultural chemicals. In addition, the solubility of the sanitary goods is improved due to the dextrin treatment. As a result, the sanitary goods are superior not only in sterilization and disinfection effect but also in masking effect against foreign odor. The present invention has such extremely useful effects on sanitation or the like.

## Claims

1. Catechin containing sanitary goods, wherein said sanitary goods are impregnated with organic catechin extracted with purified water from tea leaves grown organically without agricultural chemicals, and subject to dextrin treatment

2. Catechin containing sanitary goods according to Claim 1 wherein the tea leaves grown organically without agricultural chemicals are tea leaves grown in Sri Lanka.
